# EUROPÄISCHE PATENTSCHRIFT

(11) **EP 1 372 634 B1**
(45) Veröffentlichungstag und Bekanntmachung des Hinweises auf die Patenterteilung: **11.11.2009**
(21) Anmeldenummer: 02729423.0
(22) Anmeldetag: 08.01.2002
(51) Int. Cl.: A61K 31/197, A61K 31/225, A61P 1/04, A61P 1/16, A61P 9/00, A61P 9/14

(54) **VERWENDUNG VON FUMARSÄUREAMIDEN**
USE OF FUMARIC ACID AMIDES
UTILISATION D'AMIDES DE L'ACIDE FUMARIQUE

(30) Priorität: 12.01.2001 DE 10101307; 06.07.2001 DE 10133004
(43) Veröffentlichungstag der Anmeldung: 02.01.2004
(73) Patentinhaber: Biogen Idec International GmbH, 6300 Zug (CH)
(72) Erfinder: JOSHI, Rajendra, Kumar, CH-8048 Zürich (CH); STREBEL, Hans-Peter, CH-6006 Luzern (CH)
(74) Vertreter: Schwabe - Sandmair - Marx
(86) Internationale Anmeldenummer: PCT/EP2002/000107
(87) Internationale Veröffentlichungsnummer: WO 2002/055063

(56) Entgegenhaltungen:
- EP-A- 0 103 274
- WO-A-89/01930
- DE-A- 19 721 099
- DE-A- 19 853 487
- US-A- 3 832 287
- SUBASINGHE N ET AL: "SYNTHESIS OF ACYCLIC AND DEHYDROASPARTIC ACID ANALOGUES OF AC-ASP- GLU-OH AND THEIR INHIBITION OF RAT BRAIN N-ACETYLATED ALPHA- LINKED ACIDIC DIPEPTIDASE (NAALA DIPEPTIDASE)" JOURNAL OF MEDICINAL CHEMISTRY, AMERICAN CHEMICAL SOCIETY. WASHINGTON, US, Bd. 33, Nr. 10, 1990, Seiten 2734-2744, XP000652345 ISSN: 0022-2623
- DATABASE WPI Section Ch, Week 199808 Derwent Publications Ltd., London, GB; Class B05, AN 1998-086593 XP002227956 & WO 97 48400 A (GUILFORD PHARM INC), 24. Dezember 1997 (1997-12-24)
- BELLIER B ET AL: "Replacement of glycine with dicarbonyl and related moieties in analogues of the C-terminal pentapeptide of cholecystokinin: CCK(2) agonists displaying a novel binding mode." JOURNAL OF MEDICINAL CHEMISTRY. UNITED STATES 5 OCT 2000, Bd. 43, Nr. 20, 5. Oktober 2000 (2000-10-05), Seiten 3614-3623, XP008012592 ISSN: 0022-2623
- KAMIYAMA, TSUTOMU ET AL: "Ro 09-1679, a novel thrombin inhibitor" JOURNAL OF ANTIBIOTICS (1992), 45(3), 424-7 , XP001109494
- CHOO H-Y P ET AL: "Design and synthesis of alpha,beta-unsaturated carbonyl compounds as potential ACE inhibitors" EUROPEAN JOURNAL OF MEDICINAL CHEMISTRY, EDITIONS SCIENTIFIQUE ELSEVIER, PARIS, FR, Bd. 35, Nr. 6, Juni 2000 (2000-06), Seiten 643-648, XP004330455 ISSN: 0223-5234
- KRSTENANSKY, JOHN L. ET AL: "Development of MDL 28,050, a small stable antithrombin agent based on a functional domain of the leech protein, hirudin" THROMBOSIS AND HAEMOSTASIS (1990), 63(2), 208-14 , XP000283201
- LANGLOIS, M. ET AL: "Synthesis of symmetrical pseudopeptides as potential inhibitors of the human immunodeficiency virus-1 protease" EUROPEAN JOURNAL OF MEDICINAL CHEMISTRY (1994), 29(9), 639-47 , XP001109491
- HOLROYD, STEPHEN E. ET AL: "Rational design and binding of modified cell-wall peptides to vancomycin-group antibiotics: factorizing free energy contributions to binding" TETRAHEDRON (1993), 49(41), 9171-82 , XP001109490
- GRIEHL, C. ET AL: ".alpha.-Aspartyl peptides by addition of amines to N-maleylamino acid derivatives" CHEMISTRY OF PEPTIDES AND PROTEINS (1993), 5/6(PT. A), 99-103 , XP001161284
- ONDRUS, V. ET AL: "A simple synthesis of some analogs of natural antibiotics" CHEMICAL PAPERS (1997), 51(3), 164-166 , XP008020137
- GALPIN, I. J. ET AL: "The synthesis of an insulin active site analog" TETRAHEDRON (1983), 39(1), 149-58 , XP001162826
- BARRETT, ALAN J. ET AL: "L-trans-Epoxysuccinyl-leucylamido(4-guani dino)butane (E-64) and its analogs as inhibitors of cysteine proteinases including cathepsins B, H, and L" BIOCHEMICAL JOURNAL (1982), 201(1), 189-98 , XP008020148
- AMAMOTO, TOSHIRO ET AL: "Effect of E-64, thiol protease inhibitor, on the secondary anti-SRBC response in vitro" MICROBIOLOGY AND IMMUNOLOGY (1984), 28(1), 85-97 , XP000615667
- PORTOGHESE, P. S. ET AL: "Synthesis and biological activity of analogs of.beta.- chlornaltrexamine and.beta.-funaltrexamine at opioid receptors" JOURNAL OF MEDICINAL CHEMISTRY (1986), 29(10), 1861-4 , XP001162822
- BIRCH, ARTHUR J. ET AL: "Metabolites of Aspergillus indicus: the structure and some aspects of the biosynthesis of dihydrocanadensolide" AUSTRALIAN JOURNAL OF CHEMISTRY (1968), 21(11), 2775-84 , XP008020154
- GERHARD, UTE ET AL: "The free energy change of restricting a bond rotation in the binding of peptide analogs to vancomycin group antibiotics" BIOORGANIC & MEDICINAL CHEMISTRY LETTERS (1993), 3(5), 803-8 , XP008020158
- ROSSI, DOMENICO ET AL: "Approach to the use of benzylpenicillin acylase for configurational correlations of amino compounds. 2. Hydrolysis of N-(p-aminophenylacetyl) derivatives of some chiral primary amines" JOURNAL OF ORGANIC CHEMISTRY (1979), 44(13), 2222-5 , XP001162820
- DATABASE BIOSIS [Online] BIOSCIENCES INFORMATION SERVICE, PHILADELPHIA, PA, US; 1976 KURODA K ET AL: "INHIBITORY EFFECT OF CAPSELLA-BURSA-PASTORIS EXTRACT ON GROWTH OF EHRLICH SOLID TUMOR IN MICE" Database accession no. PREV197662032843 XP002189051 & CANCER RESEARCH, Bd. 36, Nr. 6, 1976, Seiten 1900-1903, ISSN: 0008-5472
- DATABASE BIOSIS [Online] BIOSCIENCES INFORMATION SERVICE, PHILADELPHIA, PA, US; 1996 SCHMIDT KERSTIN N ET AL: "Anti-psoriatic drug anthralin activates transcription factor NF-kappa-B in murine keratinocytes." Database accession no. PREV199699044855 XP002189050 & JOURNAL OF IMMUNOLOGY, Bd. 156, Nr. 11, 1996, Seiten 4514-4519, ISSN: 0022-1767
- SCHIRMEISTER TANJA: "Aziridine-2,3-dicarboxylic acid derivatives as inhibitors of papain." ARCHIV DER PHARMAZIE (WEINHEIM), Bd. 329, Nr. 5, 1996, Seiten 239-244, XP008012565 ISSN: 0365-6233

## Beschreibung

Die vorliegende Erfindung betrifft die Verwendung bestimmter Fumarsäure diamide bzw. Monoamidofumarsäuremonoester zur Herstellung eines Arzneimittels und diese Verbindungen enthaltende Arzneimittel.

Fumarsäuredialkylester sowie Fumarsäuremonoalkylester und Salze derselben werden seit langem mit Erfolg zur Behandlung der Psoriasis verwendet. Diese Verwendung ist in einer Anzahl von Patenten beschrieben worden, siehe beispielsweise die EP-A-0 188 749, DE-25 30 372, DE 26 21 214 oder EP-B-0 312 697.

Weiterhin beschrieben ist die Verwendung der Fumarsäuremono- oder -diester zur Behandlung von Autoimmunerkrankungen, wie beispielsweise der Polyarthritis, der Multiplen Sklerose (siehe DE 197 21 099.6 sowie DE 198 53 487.6), aber auch zur Verwendung in der Transplantationsmedizin (siehe DE 198 53 487.6 und DE 198 39 566.3). Aus den nicht vorveröffentlichten deutschen Anmeldungen DE 101 01 307.8 sowie DE 100 00 577.2 ist außerdem die Verwendung der Fumarsäuremono- und -diester zur Behandlung NFkappaB vermittelter Erkrankungen wie die Behandlung mitochondrialer Erkrankungen bekannt. Alle genannten Druckschriften beschreiben jedoch lediglich Fumarsäuremono- und -diester, gegebenenfalls in Form bestimmter Salze, das heißt Verbindungen, in denen eine oder beide Säurefunktionen der Fumarsäure mit einem Alkohol verestert sind.

B. Bellier et al. "Replacement of Glycine with Dicarbonyl and related Moieties in analogues of the C-terminal pentapeptide of cholecystokinin: CCK(2)agonists displaying a novel binding mode", Journal of Medicinal Chemistry, 43, 2000, 3614-23 beschreiben unter anderem bestimmte Fumarsäureamide, die Fumarsäurederivate von Neuropeptiden darstellen und deren therapeutische Verwendung.

WO89/019 A beschreibt bestimmte Fumarsäureamide, deren Ester und Salze und deren Verwendung zur Erleichterung der Symptome der Psoriasis und zur Stimulierung der Verdauung und des Appetits.

John L. Krstenanski et al. "Development of MDL 28, 050, a small stable antitrombin agent based on a functional domain of the leech protein, hirudin" Thrombosis and Haemostasis, 63(2), 1990, 208-14 beschreiben bestimmte Fumarsäure-Peptidhormonkonjugate mit 10 Aminosäuren und deren Verwendung als wirkungsvolle in in-vitro und in-vivo-Modellen der Thrombosebehandlung.

Die genannten Fumarsäureester weisen wegen deren Flüchtigkeit bzw. Sublimierbarkeit jedoch den Nachteil auf, dass sie bei der Herstellung pharmazeutischer Produkte, insbesondere in fester Form zur oralen Verabreichung, schwierig zu handhaben sind. Genauer erfordert die Herstellung dieser Produkte Schutzmaßnahmen wie die Verwendung von A-temmasken, Handschuhen, Schutzanzügen usw.

Darüber hinaus werden die Fumarsäureester nach oraler Verabreichung im Magen-DarmTrakt resorbiert und von allen Körperzellen unspezifisch aus dem Blutstrom aufgenommen. Um dennoch an bzw. in den Zielzellen einen therapeutisch wirksamen Spiegel des Wirkstoffs bereitzustellen, müssen daher entsprechend hohe Dosen verabreicht werden. Diese hohen Dosen führen wiederum zu den bekannten Nebenwirkungen der Fumarsäuretherapie wie Flush-Symptome (Rötungen) oder gastrointestinalen Reizungen (Übelkeit, Durchfälle, Blähungen). Die Nebenwirkungen können zwar, wie im oben genannten Stand der Technik beschrieben, durch die Verabreichung des Wirkstoffes in Form von Mikrotabletten erheblich gesenkt, jedoch nicht vollständig vermieden werden.

Gleichzeitig werden die Fumarsäureester im Blut rasch hydrolysiert und die Hydrolyseprodukte Alkohol und Fumarsäure bzw. Fumarsäuremonoester verstoffwechselt. Zum Erhalt therapeutisch wirksamer Spiegel ist daher eine wiederholte, häufig Verabreichung erforderlich. Zwar lässt sich bezüglich der Nebenwirkungen ein gewisser Gewöhnungseffekt beobachten, eine weitere Verringerung der Nebenwirkungsrate wäre jedoch wünschenswert.

Es ist daher Aufgabe der vorliegenden Erfindung, Fumarsäurederivate und deren Verwendung zur Verfügung zu stellen, die sich gezielt verabreichen lassen, beständiger gegenüber der Hydrolyse und einfacher zu handhaben sind.

Gelöst wird die vorliegende Aufgabe durch bestimmte Fumarsäuremono- und -diamide bzw. Monoamidofumarsäuremonoester, deren Verwendung zur Herstellung von Arzneimitteln zur Therapie bestimmter Erkrankungen und diese enthaltende Arzneimittel.

Fumarsäurediamide bzw. Monoamide sind bereits in US-A-5,242,905 und US-A-5,214,196 an Blank zur Therapie der Psoriasis beschrieben. Allerdings beschreiben die genannten Druckschriften lediglich die Herstellung einzelner Fumarsäuremono- oder -diamide. Nicht beschrieben ist die Herstellung pharmazeutischer Produkte und die Applikation der Amide am Menschen. Als theoretischen Vorteil der Fumaramide gegenüber den Fumarsäureestern nennen die Druckschriften die Bereitstellung bestimmter Aminosäuren aus den Fumaramiden in den Keratinozyten, zur Komplettierung eines defizitären Stoffwechsels bei der Psoriasis.

Die Erfinder haben nun überraschen gefunden, dass Fumarsäuremono- und -diamide bzw. Monoamidofumarsäuremonoester vorteilhaft zur Therapie einer Vielzahl von Erkrankungen eingesetzt werden können. Genauer betrifft die vorliegende Erfindung in einem ersten Aspekt derselben daher die Verwendung von Fumarsäureamiden der Formel (I) 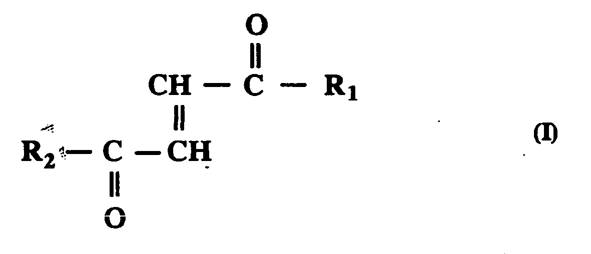 in der
R₁ für OR₃ oder für einen über eine Amidbindung gebundenen D-oder L-Aminosäurerest -NH-CHR₄-COOH steht, worin R₃ ein geradkettiger oder verzweigter Alkylrest, ausgewählt aus der Gruppe bestehend aus Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl ist, und
R₂ für einen über eine Amidbindung gebundenen D-oder L-Aminosäurerest -NH-CHR₅-COOH steht und worin R₄ und R₅ unabhängig voneinander ausgewählt sind aus der Gruppe, bestehend aus den Seitenketten von Ala, Val, Leu, Trp, Phe, Met, Tyr, Thr, Cys, Asn, Gln, Asp, Glu, Lys, Arg, His, Citrullin, Hcy, Hse, Hyp, Hyl, Orn, Sar und Me-Gly,
mit der Maßgabe, dass
dann, wenn R₁ = -NH-CHR₄-COOH ist, R₂ für -NH-CHR₅-COOH steht, worin R₅ ausgewählt ist aus der Gruppe, bestehend aus den Seitenketten von Asn, Gln, Lys, Arg oder His,
zur Herstellung eines Arzneimittels
(1) zur Therapie einer Autoimmunerkrankung ausgewählt aus der Gruppe bestehend aus der Polyarthritis, insbesondere der reumatoiden Arthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
(2) zur Verwendung in der Transplantationsmedizin (Host-versus-Graft-Reaktionen),
(3) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien sowie
(4) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata, (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis, (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen Nephritis, der Postangioplastie-Restenose, das Reperfusionssyndrom, der cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barré-Syndrom, der postherpetischen oder postzoster Neuralgie (engl.: postherpetic neuralgia), der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling (engl.: cardiac remodeling), interstitiellen Zystitis, Diabetes mellitus Typ II, der Strahlensensibilisierung maligner Tumore (engl.: human tumor radiosensitization), der Mehrfachresistenz maligner Zellen auf Chemotherapeutika (engl.: multidrug resistance in chemotherapy), Granuloma annulare und Krebserkrankungen wie Mamma Karzinom, Kolonkarzinom, Melanom, primäres Leberzellkarzinom, A-denokarzinom, Kaposi Sarkom, Prostatakarzinom, Leukämie wie der akuten myeloischen Leukämie, dem multiplen Myelom (Plasmozytom), Burkitt-Lymphom, und den Castleman-Tumor.

Mit dem Begriff "Seitenkette einer natürlichen oder synthetischen Aminosäure" sind die am α-Kohlenstoffatom der Aminosäure stehenden Reste jeder natürlichen oder synthetisch hergestellten Aminosäure gemeint. Die Aminosäurereste R₁ und R₂ können in der D- und der L-Konfiguration vorliegen. Bevorzugt ist die natürliche L-Konfiguration. Im Folgenden werden zur Kennzeichnung der Aminosäuren die üblichen Abkürzungen bzw. Bezeichnungen im Drei-Buchstaben-Code verwendet.

Gemäß einer Ausführungsform werden insbesondere Verbindung der Formel (I) verwendet bzw. beansprucht, in denen R₁ für einen L-Aminosäurerest -NH-CHR₄-COOH und R₂ für einen L-Aminosäurerest -NH-CHR₅-COOH steht, worin R₄ und R₅ gleich oder voneinander verschieden sein können und die oben angegebene Bedeutung haben.

Gemäß einer anderen Ausführungsform werden Verbindungen der Formel (I) verwendet bzw. beansprucht, in denen R₁ für -OR₃ und R₂ für einen L-Aminosäurerest -NH-CHR₅-COOH steht, worin R₅ ausgewählt ist aus der Gruppe, bestehend aus den Seitenketten von Ala, Val, Leu, Trp, Phe, Met, Tyr, Thr, Cys, Asn, Gln, Asp, Glu, Lys, Arg, His, Citrullin, Hcy, Hse, Hyp, Hyl, Orn, Sar, und Me-Gly.

Ganz besonders bevorzugt handelt es sich bei R₅ in beiden Ausführungsformen um eine polare Aminosäure, noch stärker bevorzugt um eine Ladung tragende Aminosäure, ausgewählt aus der Gruppe bestehend aus Asparagin, Glutamin, Lysin, Arginin und Histidin.

Wenn der Rest R₁ -OR₃ bedeutet, d.h. die erfindungsgemäß zu verwendende bzw. beanspruchte Verbindung der Formel (I) ein Monoamidofumarsäuremonoester ist, ist R₃ ausgewählt aus der Gruppe, bestehend aus Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl,Octyl. Am meisten bevorzugt ist R₃ Methyl oder Ethyl.

Die erfindungsgemäßen Fumarsäureamide können gemäß der oben genannten US-Patente von Blank hergestellt werden.

In einem weiteren Aspekt offenbart die vorliegende Beschreibung Arzneimittel, enthaltend ein wie oben definiertes Fumarsäureamid oder Gemische derselben. Die über die erfindungsgemäße Verwendung und/oder durch den Einsatz der beschriebenen Fumarsäureamide erhaltenen Arzneimittel können für die orale, nasale, parenterale, rectale, pulmonale, ophthale oder transdermale Verabreichung geeigneter Form vorliegen.

Bevorzugt ist das erfindungsgemäß erhaltene Arzneimittel zur oralen Verabreichung bestimmt und liegt in Form von Tabletten, Dragees, Kapseln, Granulat, Trinklösungen, Liposomen, Nanokapseln, Mikrokapseln, Mikrotabletten, Pellets oder Pulvern sowie in Kapseln gefülltem Granulat, in Kapseln gefüllten Mikrotabletten, in Kapseln gefüllten Pellets oder in Kapseln gefülltem Pulver vor.

Gemäß einer besonders bevorzugten Ausführungsform ist das erfindungsgemäß erhaltene Arzneimittel eine feste orale Dosisform und weist dann noch bevorzugter einen magensaftresistenten Überzug auf. Beispielsweise kann dieser Überzug auf Tabletten, Dragees, Mikrotabletten, Pellets oder Kapseln vorgesehen sein.

Grundsätzlich kann das erfindungsgemäß erhaltene Arzneimittel geeignete, pharmazeutisch annehmbare Träger, Hilfsstoffe, Zusatzstoffe usw. enthalten. Diese sind dem Fachmann bekannt und bedürfen keiner Erläuterung.

Am meisten bevorzugt ist die Verwendung von Mikrotabletten oder Pellets. Bevorzugt weisen diese ohne Beschichtung einen mittleren Durchmesser von 300 bis 5000 µm, stärker bevorzugt 300 bis 2000 µm auf.

Bei parenteraler Verabreichung über Injektion liegt die Zubereitung in hierfür geeigneter Form vor. Es können alle üblichen flüssigen, für die Injektion geeigneten Träger verwendet werden.

In jedem Fall ist bevorzugt, dass das erfindungsgemäß erhaltene Arzneimittel pro Einzeldosis eine Menge des Fumarsäureamids der Formel (I) enthält, die einer Menge von 1 bis 500 mg, vorzugsweise 10 bis 300 mg und am meisten bevorzugt 10 bis 200 mg Fumarsäure entspricht bzw. äquivalent ist.

## Patentansprüche

1. Verwendung von Fumarsäureamiden der Formel (I) 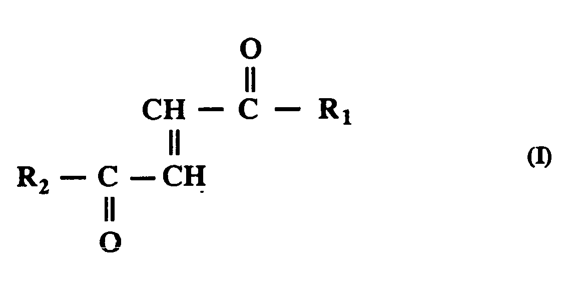 in der
R₁ für OR₃ oder für einen über eine Amidbindung gebundenen D- oder L-Aminosäurerest -NH-CHR₄-COOH steht, worin R₃ ein geradkettiger oder verzweigter Alkylrest, ausgewählt aus der Gruppe bestehend aus Methyl, Ethyl, n-Propyl, Isopropyl, n-Butyl, sec-Butyl, t-Butyl, Pentyl, Cyclopentyl, 2-Ethylhexyl, Hexyl, Cyclohexyl, Heptyl, Cycloheptyl, Octyl ist, und
R₂ für einen über eine Amidbindung gebundenen D- oder L-Aminosäurerest -NH-CHR₅-COOH steht und worin R₄ und R₅ unabhängig voneinander ausgewählt sind aus der Gruppe, bestehend aus den Seitenketten von Ala, Val, Leu, Trp, Phe, Met, Tyr, Thr, Cys, Asn, Gln, Asp, Glu, Lys, Arg, His, Citrullin, Hcy, Hse, Hyp, Hyl, Orn, Sar und Me-Gly,
mit der Maßgabe, dass
dann, wenn R₁ = -NH-CHR₄-COOH ist, R₂ für -NH-CHR₅-COOH steht, worin R₅ ausgewählt ist aus der Gruppe, bestehend aus den Seitenketten von Asn, Gln, Lys, Arg oder His,
zur Herstellung eines Arzneimittels
(1) zur Therapie einer Autoimmunerkrankung ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis,
(2) zur Verwendung in der Transplantationsmedizin,
(3) zur Therapie mitochondrialer Erkrankungen, ausgewählt aus der Gruppe bestehend aus dem Parkinson-Syndrom, der Alzheimer-Krankheit, der Chorea-Huntington-Krankheit, der Retinopathia pigmentosa oder mitochondrialen Enzephalomyopathien, sowie
(4) zur Therapie von NFkappaB vermittelten Erkrankungen, ausgewählt aus der Gruppe, bestehend aus der progressiven systemischen Sklerodermie, der Osteochondritis syphilitica (Wegener's Disease), der Cutis marmorata (Livedo Reticularis), der Behcet-Disease, Panarteritis, Colitis ulcerosa, Vasculitis, der Osteoarthritis, Gicht, Ateriosklerosis, der Reiter's Erkrankung, der bronchozentischen Granulomatose, Encephalitis-Typen, dem Endotoxin-Schock (septisch-toxischer Schock), der Sepsis, der Pneumonie, der Encephalomyelitis, der Anorexia nervosa, der Hepatitis (der akuten Hepatitis, der chronischen Hepatitis, der toxischen Hepatitis, der Alkoholhepatitis, der viralen Hepatitis, der Gelbsucht, der Leberinsuffizienz und der cytomegaloviralen Hepatitis), der Rennert T Lymphomatosis, der mesangialen Nephritis, der Postangioplastie-Restenose, das Reperfusionssyndrom, der cytomegaloviralen Retinopathie, Adenoviralen Erkrankungen wie adenoviralen Erkältungserkrankungen, dem adenoviralen Pharyngoconjunctivalfieber, und der adenoviralen Ophthalmie, AIDS, dem Guillain-Barré-Syndrom, der postherpetischen oder postzoster Neuralgie, der inflammatorischen demyelinisierenden Polyneuropathie, der Mononeuropathia multiplex, der Mukoviszidose, Morbus Bechterew, Barett-Ösophagus, EBV-(Epstein-Barr-Virus)-Infektion, dem kardialen Remodeling, interstitiellen Zystitis, Diabetes mellitus Typ II, der Strahlensensibilisierung maligner Tumore, der Mehrfachresistenz maligner Zellen auf Chemotherapeutika, Granuloma annulare und Krebserkrankungen wie Mamma Karzinom, Kolonkarzinom, Melanom, primäres Leberzellkarzinom, Adenokarzinom, Kaposi Sarkom, Prostatakarzinom, Leukämie wie der akuten myeloischen Leukämie, dem multiplen Myelom (Plasmozytom), Burkitt-Lymphom, und den Castleman-Tumor.

2. Verwendung nach Anspruch 1, worin R₁ für -OR₃ und R₂ für einen L-Aminosäurerest -NH-CHR₅-COOH steht.

3. Verwendung nach Anspruch 1, worin R₁ für einen L-Aminosäurerest -NH-CHR₄-COOH und R₂ für einen L-Aminosäurerest -NH-CHR₅-COOH steht.

4. Verwendung nach Anspruch 1 oder 2, worin R₃ Methyl oder Ethyl ist.

5. Verwendung nach Anspruch 1. 2 oder 3, worin R₅ ausgewählt ist aus der Gruppe, bestehend aus den Seitenketten von Gln, Asn, Lys, Arg, His.

6. Verwendung nach einem der Ansprüche 1 bis 5, wobei das Arzneimittel in für die orale, nasale, parenterale, rectale, pulmonale, ophthale oder transdermale Verabreichung geeigneter Form vorliegt.

7. Verwendung nach einem der Ansprüche 1 bis 5, wobei das Arzneimittel zur oralen Verabreichung bestimmt ist und in Form von Tabletten, Dragees, Kapseln, Granulat, Trinklösungen, Liposomen, Nanokapseln, Mikrokapseln, Mikrotabletten, Pellets oder Pulvern sowie in Kapseln gefülltem Granulat, Mikrotabletten, Pellets oder Pulver vorliegt.

8. Verwendung nach Anspruch 7, worin das Arzneimittel eine feste orale Dosisform ist und einen magensaftresistenten Überzug aufweist.

9. Verwendung nach Anspruch 7, worin die Mikrotabletten oder Pellets ohne Beschichtung einen mittleren Durchmesser von 300-5000 µm aufweisen, vorzugsweise 300-2000 µm.

10. Verwendung nach einem der Ansprüche 1 bis 9, wobei das Arzneimittel pro Einzeldosis eine Menge des Fumarsäureamids der Formel (I) enthält, die 1 bis 500 mg, vorzugsweise 10 bis 300 mg, Fumarsäure entspricht.

## Claims

1. Use of fumaric acid amides of the formula (I) 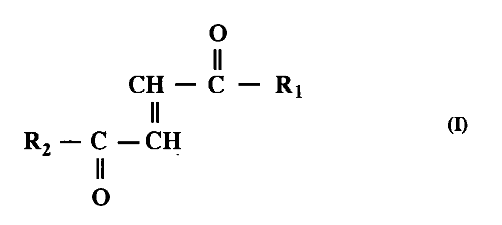 wherein
R₁ represents -OR₃ or a D- or L-amino acid radical -NH-CHR₄-COOH bonded via an amide bond, wherein R₃ is a straight-chained or branched alkyl radical,
selected from the group consisting of methyl, ethyl, n-propyl, isopropyl, n-butyl, sec-butyl, t-butyl, pentyl, cyclopentyl, 2-ethylhexyl, hexyl, cyclohexyl, heptyl, cycloheptyl, octyl, and
R₂ represents a D- or L-amino acid radical -NH-CHR₅-COOH bonded via an amide bond, wherein each of R₄ and R₅ is independently selected from the group, consisting of the side chains of Ala, Val, Leu, Trp, Phe, Met, Tyr, Thr, Cys, Asn, Gln, Asp, Glu, Lys, Arg, His, Citrullin, Hcy, Hse, Hyp, Hyl, Orn, Sar and Me-Gly,
with the proviso that
when R₁ = -NH-CHR₄-COOH, R₂ represents -NH-CHR₅-COOH, wherein R₅ is selected from the group consisting of the side chains of Asn, Gln, Arg, or His
for manufacturing a medicament
(1) for the therapy of an autoimmune disease selected from the group consisting of polyarthritis, multiple sclerosis, graft-versus-host reactions, juvenile-onset diabetes, Hashimoto's thyroiditis, Grave's disease, systemic Lupus erythematodes, Sjogren's syndrome, pernicious anaemia and chronic active (= lupoid) hepatitis;
(2) for the therapy or use in transplantation medicine;
(3) for the therapy of mitochondrial diseases selected from the group consisting of Parkinson syndrome, Alzheimer's disease, Chorea Huntington disease, retinopathia pigmentosa or forms of mitochondrial encephalomyopathy; or
(4) for the therapy of an NF-kappaB mediated disease selected from the group consisting of progressive systemic sclerodermia, osteochondritis syphilitica (Wegener's disease), cutis marmorata (*livedo reticularis),* Behcet disease, panarteriitis, colitis ulcerosa, vasculitis, osteoarthritis, gout, arteriosclerosis, Reiter's disease, pulmonary granulomatosis, types of encephalitis, endotoxic shock (septic-toxic shock), sepsis, pneumonia, encephalomyelitis, anorexia nervosa, hepatitis (acute hepatitis, chronic hepatitis, toxic hepatitis, alcohol hepatitis, viral hepatitis, jaundice, hepatic insufficiency cytomegaloviral hepatitis), Rennert T-lymphomatosis, mesangial nephritis, post-angioplastic restenosis, reperfusion syndrome, cytomegaloviral retinopathy, adenoviral diseases such as adenoviral colds, adenoviral pharyngoconjunctival fever and adenoviral ophthalmia, AIDS, Guillain-Barré syndrome, post-herpetic or post-zoster neuralgia, inflammatory demyelinising polyneuropathy, mononeuropathia multiplex, mucoviscidosis, Bechterew's disease, Barett oesophagus, EBV (Epstein-Barr virus) infection, cardiac remodeling, interstitial cystitis, diabetes mellitus type II, human tumour radiosensitisation, multi-resistance of malignant cells to chemotherapeutic agents, granuloma annulare and cancers such as mamma carcinoma, colon carcinoma, melanoma, primary liver cell carcinoma, adenocarcinoma, Kaposi's sarcoma, prostate carcinoma, leukaemia such as acute myeloid leukaemia, multiple myeloma (plasmocytoma), Burkitt lymphoma and Castleman tumour.

2. The use according to claim 1 wherein R₁ represents -OR₃ and R₂ represents an L-amino acid radical -NH-CHR₅-COOH.

3. The use according to claim 1, wherein R₁ represents an L-amino acid radical -NH-CHR₄-COOH and R2 represents -NH-CHR₅-COOH.

4. The use according to claim 1 or 2, wherein R₃ is methyl or ethyl.

5. The use according to claim 1, 2 or 3, wherein R₅ is selected from the group consisting of Gln, Asn, Lys, Arg, His.

6. The use according to any one of claims 1 to 5, wherein said medicament is in a form suitable for oral, nasal, parenteral, rectal, pulmonal, ophthal or transdermal administration.

7. The use according to any one of claims 1 to 5, said medicament being intended for oral administration and being present in the form of tablets, coated tablets, capsules, granulate, solutions for drinking, liposomes, nano-capsules, micro-capsules, micro-tablets, pellets or powders; or in the form of granulate, micro-tablets, pellets or powder filled in capsules.

8. The use according to claim 8, said medicament being a solid oral dosage form and having an enteric coating.

9. The use according to claim 7, wherein the micro-tablets or pellets without coating have a mean diameter of 300 to 5000 µm, preferably 300 to 2000 µm.

10. The use according to any of the claims 1 to 9 wherein said medicament contains an amount of said fumaric acid amide of formula (I) corresponding to 1 to 500 mg, preferably 10 to 300 mg of fumaric acid per single dose.

## Revendications

1. Utilisation d'amides de l'acide fumarique de formule (I) 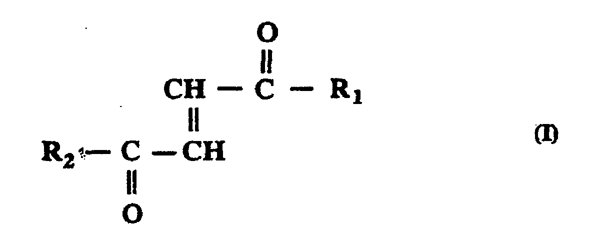 dans laquelle
R₁ représente OR₃ ou un radical aminoacide -NH-CHR₄-COOH D ou L lié par une liaison amide, R₃ étant un radical alkyle linéaire ou ramifié choisi parmi le groupe consistant en le méthyle, l'éthyle, le n-propyle, l'isopropyle, le n-butyle, le sec.-butyle, le t-butyle, le pentyle, le cyclopentyle, le 2-éthylhexyle, l'hexyle, le cyclohexyle, l'heptyle, le cycloheptyle, l'octyle, et
R₂ représente un radical aminoacide -NH-CHR₅-COOH D ou L lié par une liaison amide, R₄ et R₅ étant choisis indépendamment l'un de l'autre parmi le groupe consistant en les chaînes latérales de Ala, Val, Leu, Trp, Phe, Met, Tyr, Thr, Cys, Asn, Gln, Asp, Glu, Lys, Arg, His, citrulline, Hcy, Hse, Hyp, Hyl, Orn, Sar et Me-Gly,
avec la condition que,
quand R₁ est = -NH-CHR₄-COOH, R₂ représente -NH-CHR₅-COOH, R₅ étant choisi parmi le groupe consistant en les chaînes latérales de Asn, Gln, Lys, Arg ou His,
pour la préparation d'un médicament
(1) pour la thérapie d'une maladie auto-immune choisie parmi le groupe consistant en la polyarthrite, la sclérose en plaques, la réaction dite graft-versus-host, le diabète juvénile, la thyroïdite d'Hashimoto, le Grave's disease (maladie de Basedow), le lupus érythémateux disséminé (LED), le syndrome de Sjögren, l'anémie pernicieuse et l'hépatite chronique active (= lupoïde),
(2) pour une application dans la médecine des greffes,
(3) pour la thérapie d'affection mitochondriales, choisies parmi le groupe consistant en le syndrome de Parkinson, la maladie d'Alzheimer, la chorée de Huntington, la rétinopathie pigmentaire ou les encéphalomyopathies mitochondriales, ainsi que
(4) pour la thérapie d'affections transmises par le facteur NF-kappa B, choisies parmi le groupe consistant en la sclérodermie systémique progressive, l'ostéochondrite syphilitique (Wegener's Disease), la Cutis Marmorata (Livedo reticularis), la maladie de Behçet, la panartérite, la colite ulcéreuse, la vasculite, l'ostéoarthrite, la goutte, l'athérosclérose, la maladie de Reiter, la granulomatose broncho-centrique, des types d'encéphalite, le choc endotoxique (choc septique et toxique), la septicémie, la pneumonie, l'encéphalomyélite, l'anorexie nerveuse, l'hépatite (hépatite aiguë, hépatite chronique, hépatite toxique, hépatite alcoolique, hépatite virale, jaunisse, insuffisance hépatique et hépatite cytomégalovirale), la lymphomatose T de Rennert, la néphrite mésangiale, la resténose post-angioplastique, le syndrome de réirrigation, la rétinopathie cytomégalovirale, les maladies à adénovirus ainsi que les rhumes à adénovirus, la fièvre pharyngo-conjonctivale à adénovirus, et l'ophtalmie à adénovirus, le SIDA, le syndrome de Guillain-Barré, la névralgie post-herpétique ou post-zostérienne, la polynévropathie démyélisante inflammatoire, la mononévropathie multiplexe, la mucoviscidose, le morbus Bechterew, l'oesophagite de Barett, l'infection EBV (Epstein-Barr-Virus), le remodelage cardiaque, la cystite interstitielle, le diabète sucré de type II, la sensibilisation aux radiations de tumeurs malignes, la multirésistance des tumeurs malignes aux agents chimiothérapeutiques, le granulome annulaire et les affections cancéreuses comme le cancer du sein, le cancer du côlon, le mélanome, le carcinome primaire des cellules hépatiques, l'adénocarcinome, le sarcome de Kaposi, le carcinome de la prostate, la leucémie ainsi que la leucémie myéloïque, le myélome multiple (plasmocytome), le lymphome de Burkitt et la tumeur de Castleman.

2. Utilisation selon la revendication 1, dans laquelle R₁ représente -OR₃ et R₂ un radical aminoacide L -NH-CHR₅-COOH.

3. Utilisation selon la revendication 1 ou 2, dans laquelle R₁ représente un radical aminoacide L -NH-CHR₄-COOH et R₂ un radical aminoacide L -NH-CHR₅-COOH.

4. Utilisation selon la revendication 1 ou 2, dans laquelle R₃ est un méthyle ou un éthyle.

5. Utilisation selon la revendication 1, 2 ou 3, dans laquelle R₅ est choisi parmi le groupe consistant en les chaînes latérales de Gln, d'Asn, de Lys, d'Arg, d'His.

6. Utilisation selon l'une quelconque des revendications 1 à 5, dans laquelle le médicament est présent sous une forme appropriée pour une administration orale, nasale, parentérale, rectale, pulmonaire, ophtalmique ou transdermale.

7. Utilisation selon l'une quelconque des revendications 1 à 5, dans laquelle le médicament est destiné à une administration orale et se présente sous la forme de comprimés, de dragées, de capsules, de granulés, de solutions buvables, de liposomes, de nanocapsules, de microcapsules, de micro-comprimés, de pastilles ou de poudre, ainsi que de granulés, de micro-comprimés, de pastilles ou de poudre remplis dans des capsules.

8. Utilisation selon la revendication 7, dans laquelle le médicament est une forme orale dosée solide et comporte une enveloppe qui résiste au suc gastrique.

9. Utilisation selon la revendication 7, dans laquelle les micro-comprimés ou les pastilles sans enveloppe présentent un diamètre moyen de 300 à 5000 µm, de préférence de 300 à 2000 µm.

10. Utilisation selon l'une quelconque des revendications 1 à 9, dans laquelle le médicament contient par dose unitaire une quantité de l'amide de l'acide fumarique de formule (I) qui correspond à 1 à 500 mg, de préférence 10 à 300 mg d'acide fumarique.
